# EUROPÄISCHE PATENTSCHRIFT

(11) **EP 0 665 435 B1**
(45) Veröffentlichungstag und Bekanntmachung des Hinweises auf die Patenterteilung: **13.09.2000**
(21) Anmeldenummer: 95100880.4
(22) Anmeldetag: 24.01.1995
(51) Int. Cl.: G01N 33/86, C12Q 1/56

(54) **Verfahren zur einfacheren Reinigung von Messgefässen bei der Bestimmung von Blutgerinnungsparametern**
Procedure for simplifying the cleaning of reaction containers used for the determination of blood clotting factors
Procédure pour simplifier le nettoyade des cuvettes de réaction utilisées pour la détermination des facteurs de coagulaiton sanguine

(30) Priorität: 29.01.1994 DE 4402631
(43) Veröffentlichungstag der Anmeldung: 02.08.1995
(73) Patentinhaber: Roche Diagnostics GmbH, 68298 Mannheim (DE)
(72) Erfinder: Adema, Enno, Dr., D-82327 Tutzing (DE); Gebert, Ulrike, D-82402 Seeshaupt (DE); Herz, Reinhard, D-82343 Poecking/Possenhofen (DE)

(56) Entgegenhaltungen:
- EP-A- 0 472 205
- DATABASE WPI Section Ch, Week 8721 Derwent Publications Ltd., London, GB; Class B04, AN 87-149480 XP002001816 & SU-A-1 262 379 (KIEV MED INST) , 7.Oktober 1986
- AMERICAN JOURNAL OF VETERINARY RESEARCH, Bd. 53, Nr. 5, Mai 1992, CHICAGO, ILL, Seiten 695-705, XP000195966 COYNE ET AL.: "Pharmacologic evaluation of factor XIIIa-like enzyme ...."
- GERNOT PETER: 'Lehrbuch der Klinischen Chemie', 1982, EDITION MEDIZIN, BASEL * Seite 370 - Seite 372 *

## Beschreibung

Die Erfindung betrifft ein Verfahren zur Bestimmung von Gerinnungsparametern unter Zusatz von Inhibitoren für die Aktivierung von Faktor XIII, Faktor XIIIₐ oder die fibrinvernetzende Wirkung von Faktor XIIIₐ.

Die Bestimmung von Gerinnungsparametern in Probenmaterial läuft üblicherweise über eine Reaktionskaskade (vgl. Haemostasis 20 (suppl. 1) 14 - 29, 1990). ab. Bei einer Vielzahl von Bestimmungen ist der letzte Schritt eine durch Thrombin katalysierte Bildung eines Fibrinclots. Auf diese Weise können beispielsweise PT (Prothrombinzeit), PTTa (partielle Thromboplastinzeit aktiviert), Fibrinogen, Faktor II, V, VII, VIII, IX, X, XI, XII, Protein C, Protein S und APC-Resistenz bestimmt werden (siehe beispielsweise Bergmeyer (Editor) (1988): Methods of Enzymology, Vol V,3rd Edition; Guglumone und Vides (1992): Thromb Haemostas 67, 46-9; Preda et al (1990): Thromb Res 60, 19-32 oder Dahlbäck et al (1993): Proc Natl. Acad. Sci. USA 90, 1004-8).

Meßgefäße zur Bestimmung von Gerinnungsparametern müßen äußerst gründlich gereinigt sein um Meßfehler zu verhindern (Gernot Peter: Lehrbuch der Klinischen Chemie, 1982, edition Medizin, Basel, Seiten 370 - 372).

Da der gebildete Fibrinclot in der Praxis nicht ohne erheblichen manuellen Aufwand aus den Meßgefäßen entfernt werden kann, werden die Meßgefäße bei der Bestimmung von Gerinnungsparametern üblicherweise nur einmal benutzt und anschließend weggeworfen. Abgesehen von den hierdurch verursachten Kosten sind solche Tests nicht applizierbar auf Geräte, an denen Küvetten mehrmals benutzt werden. Spezielle, mit Einmal-Küvetten ausgestattete, Gerinnungsanalyzer sind relativ teuer, da sie nur in geringer Stückzahl produziert werden.

Diese Nachteile beseitigt die Erfindung.

Die Erfindung besteht in einem Verfahren zur Bestimmung von Gerinnungsparametern in Probenmaterial über eine Reaktionskaskade, wobei eine von Thrombin katalysierte Bildung eines Fibrinclots erfolgt, und die Bildung des Fibrinclots gemessen wird, dadurch gekennzeichnet, daß die Bestimmung in Gegenwart eines Inhibitors für die Aktivierung von Faktor XIII, Faktor XIIIₐ oder die fibrinvernetzende Wirkung von Faktor XIIIₐ durchgeführt wird.

Wenn die Bestimmung eines Gerinnungsparameters in Gegenwart eines solchen Inhibitors durchgeführt wird, ist es anschließend möglich, den Fibrinclot unter Bedingungen, die die gegenseitigen Affinität der Fibrinmoleküle aufheben, auszulösen und, ohne weitere Bestimmungen von Gerinnungsparametern negativ zu beeinflussen, aus dem Meßgefäß durch Spülvorgänge zu beseitigen.

Potentiell ist jeder Inhibitor für die Aktivierung von Faktor XIII, Faktor XIIIₐ selbst oder die fibrinvernetzende Wirkung von Faktor XIIIₐ geeignet.

Dies sind beispielsweise: Hydroxylamine, Benzothiophen, Jodessigsäure, N-Ethylmaleinimid, Lanthanid-Ionen, Cefuroxime, L-Arginine, L-Lysine, Peptidfragmente von Faktor XIIIₐ oder Fibrin sowie Antikörper. In dem folgenden Stand der Technik sind Beispiele für diese Inhibitoren Beschrieben:
Feddersen J., Gormsen J.(1976): Thromb. Haemostasis 36
Achyuthan, E.A., Enghild, J.J., Greenberg C.S., (1991): Thromb. Haem. 65, 901
Achyuthan, E. A. Mary and C. S. Greenberg (1989): Biochem. J. 257: 331-8
Glover C.J., L.V. McIntire, C.H. Brown and E.A. Natelson (1975): J. Lab. Clin. Med. 86: 644-56
Jiang, S.T. and J.J. Lee (1992): J. Agr. Food. Chem. 40: 1101-7
Lee, K.N., L. Fesus S,T, Yanbcey, J.E. Girard and S.I. Chung (1985): J. Biol. chem. 260: 14 689-94
Samara, M., J. Soria, C. Soria, M. Maamer and A.M. Otero (1979):Prog. Chem. Fibrinnolysis Thrombolysis 4: 235-40.
Lukacova, D., Matsueda, G.R., Haber, E. and Reed, G.L.(1991): Biochemistry 30: 10164-70
Coysne C.P., Smith, J.E. and De Bowers R. M.(1992): Am J. Vet. Res 53:695-705
Achyuthan, K.E., Slaughter, T.F., Santiago M.A., Enghild, J.J. and Greenberg, C.S.(1993):
J. Biol. Chem 268: 21284-92.

Für das Auflösen des Gerinnsels ist jede Lösung geeignet, die die gegenseitige Affinität der Fibrinmoleküle aufhebt.

Dies sind beispielsweise Säuren oder Basen, Lösungen von chaotrophen Ionen wie KSCN oder Na-Salicylat, Harnstoff oder Arginine.

Die Inhibitoren werden in einer solchen Konzentration zugesetzt, daß durch Zugabe der Reinigungslösung, wie z. B. 1 M-Salzsäure, der Clot sich innerhalb max. 5 - 10 Minuten so gelöst hat, daß er vollständig aus dem Meßgefäß entfernbar ist. Für Hydroxylamine, Jodessigsäuren N-Ethylmaleinimid liegt dieser bevorzugte Konzentrationsbereich zwischen 0,5 und 20 mmol/l.

Zur Entfernung des Fibrinclots aus dem Meßgefäß wird vorzugsweise mit einer sauren Lösung (pH < 2, vorzugsweise ≤ 1) zwischen 10 Sekunden und 10 Minuten inkubiert. Vorzugsweise wird hierfür Salzsäure verwendet. Anschließend wird mit Wasser oder wäßriger Pufferlösung ein- bis dreimal gespült. Danach ist der Fibrinclot soweit entfernt, daß weitere Bestimmungen von Gerinnungsparametern nicht gestört werden.

Alternativ zu einer sauren Lösung kann auch eine der oben angegebenen Substanzen allein oder in Kombination eingesetzt werden.

### Beispiel 1

### Durchführung einer PT-Bestimmung am Meßgerät Hitachi 717

### Probe:

20 µl Citratplasma (enthält ca. 5,5 g/l Fibrinogen)

### Reagenz 1 (R1):

300 µl 100 mmol/l Trispuffer, pH 7,0
10 mmol/l Kalziumchlorid
2,5 mmol/l Hydroxylamin
3,75 mg/ml Kaninchenhirn Thromboplastin

### Reagenz 2 (R2):

300 µl, 1 mol/l HCl

Die PT-Bestimmung wird durchgeführt, indem 20 µl Plasma und 300 µl R1 gemischt werden und die Zeit bis zur Entstehung des Gerinnsels gemessen wird.

Nach der Bildung des Gerinnsels werden 300 µl R2 zugegeben, bei 37°C gemischt und nach weiteren 100 Sekunden - 10 Minuten ausgesaugt. Es wird ein- bis dreimal mit Wasser gewaschen.

Die Überprüfung der Extinktion im Meßgefäß nach Durchführung der Bestimmung und Reinigung des Meßgefäßes mit Salzsäure/Wasser ergab, daß die Extinktion des Leerwertes vor und nach der Bestimmung sich nicht signifikant unterscheidet (max. ca. 0,1 mE aus 50 Messungen).

### Beispiel 2

Zur Überprüfung, welche Substanzen als Inhibitor für Faktor XIIIₐ-Aktivität geeignet sind, wird eine PT-Bestimmung an einem Koagulometer der Fa. H. Amelung, Lemgo, DE (Typ KC 4A) durchgeführt (100 µl Plasma + 200 µl Neoplastin® Plus-Reagenz). Zwei Minuten nach dem erfolgten Clotting werden 200 µl 1 mol/l Salzsäure zugegeben und die Zeit, bis sich die Kugel wieder frei bewegen kann, gemessen. Die Ergebnise zeigt Tabelle 1.

**Tabelle 1**

| Substanz | Konzentration (mM) | Gerinnungszeit 100 %, s) | (PT Kugel bewegt nach ? min |
|---|---|---|---|
| | | | |
| Hydroylamin | 0.8 | 11.6 | 3.75 |
| | 1.7 | 12.2 | 1.75 |
| | 2.5 | 12.7 | 1.5 |
| | 3.3 | 13.4 | 1.5 |
| Jodessigsäure | 0.8 | 11.7 | 2.25 |
| | 1.7 | 12.4 | 2.25 |
| | 2.5 | 13.3 | 1.75 |
| | 3.3 | 14.2 | 1.5 |
| N-Ethylmaleinimid | 2.5 | 12.2 | > 5 |
| | 3.3 | 12.5 | 2.25 |
| | 6.6 | 12.7 | 1.5 |
| | 10 | 13.2 | 1.75 |
| | 13.3 | 13.3 | 2 |

### Beispiel 3:

Zur Überprüfung, welche Lösungen geeignet sind, den Clot nach der Messung aufzulösen, wird eine PT-Bestimmung an einem Koagulometer der F. H. Amelung, Lemgo, D, Typ (KC 4A) durchgeführt (100 µl Plasma + 200 µl Neoplastin®-Plus + Hydroxylamine, Endkonzentration 2,5 mM). Zwei Minuten nach dem erfolgten Clotting werden 200 µl Auflösemittel zugegeben und die Zeit, bis sich die Kugel wieder frei bewegen kann, gemessen. Die Ergebnisse zeigt Tabelle 2.

**Tabelle 2**

| Auflösemitel | | Kugel bewegt sich nach ? Sekunden |
|---|---|---|
| 1 M HCl | | 75 |
| 100 mM Citrat, | pH=2 | >300 |
| | pH=3 | >300 |
| | pH=4 | >300 |
| | pH=5 | >300 |
| 100 mM Glycin | pH=10 | >300 |
| | pH=11 | >300 |
| | pH=12 | >300 |
| | pH=13 | >300 |
| 1 M NaOH | | 60 |
| 2 M Na Salicylat+50 mM EDTA | pH=10 | >300 |
| | pH=11 | >300 |
| | pH=12 | >300 |
| | pH=13 | 100 |

### Beispiel 4:

### Durchführung einer PTTa Bestimmung

### Die Übertragbarkeit der für die PT-Bestimmung erhobenen Daten wurde am Koagulometer KC-4A wie folgt validiert:

10 µl 100 mM Hydroxylamin
100 µl Plasma
100 µl PTTa Reagenz (Boehringer Manheim GmbH)
3 Minuten Inkubation
100 µl CaCl₂
Messung der Gerinnungszeit
2 Minuten zusätzliche Inkubation
200 µl 1 M HCl
Messung der Zeit bis die Kugel sich wieder frei bewegt.

Die Ergebnisse sind in der Tabelle 3 dargestellt.

**Tabelle 3**

| | Gerinnungszeit (Sekunden) | Kugel bewegt sich nach ? Sekunden |
|---|---|---|
| ohne Hydroxylamin | 33,5 | > 300 |
| mit Hydroxylamin | 34,5 | 30 |

### Beispiel 5:

### Durchführung einer Protein C-Bestimmung

### Die Übertragbarkeit der für die PT-Bestimmung erhobenen Daten wurde am Koagulometer KC-4A wie folgt validiert:

10 µl 100 mM Hydroxylamin
10 µl Plasma
90 µl Owrens-Puffer (Boehringer Mannheim)
100 µl R1 Protein C Reagenz (Boehringer Mannheim)
100 µl R2 Protein C Reagens (Boehringer Mannheim)
3 Minuten Inkubation
100 µl CaCl₂-Lösung
Messung der Gerinnungszeit
2 Minuten zusätzliche Inkubation
200 µl 1 M HCl
Messung der Zeit bis die Kugel sich wieder frei bewegt

Die Ergebnisse sind in der Tabelle 4 dargestellt.

**Tabelle 4**

| | Gerinnungszeit (Sekunden) | Kugel bewegt sich nach ? Sekunden |
|---|---|---|
| ohne Hydroxylamin | 127 | > 300 |
| mit Hydroxylamin | 133 | 120 |

### Beispiel 6:

### Durchführung einer Thrombinzeit-Bestimmung

### Die Übertragbarkeit der für die PT-Bestimmung erhobenen Daten wurde am Koagulometer KC-4A wie folgt validiert:

20 µl 100 mM Hydroxylamin
200 µl Plasma
2 Minuten Inkubation
200 µl Thrombin-Reagenz (Boehringer Mannheim, Bestell-Nr. 126 594)
Messung der Gerinnungszeit
2 Minuten zusätzliche Inkubation
300 µl 1 M HCl
Messung der Zeit bis die Kugel sich wieder frei bewegt

Die Ergebnisse sind in der Tabelle 5 dargestellt.

**Tabelle 5**

| | Gerinnungszeit (Sekunden) | Kugel bewegt sich nach ? Sekunden |
|---|---|---|
| ohne Hydroxylamin | 17,3 | > 300 |
| mit Hydroxylamin | 17.1 | 80 |

## Patentansprüche

1. Verfahren zur Bestimmung von Gerinnungsparametern in Probenmaterial, wobei eine von Thrombin katalysierte Bildung eines Fibrinclots erfolgt und die Bildung des Fibrinclots gemessen wird, dadurch gekennzeichnet, daß die Bestimmung in Gegenwart eines Inhibitors für die Aktivierung von Faktor XIII, Faktor XIIIₐ selbst, oder die fibrinvernetzende Wirkung von Faktor XIIIₐ durchgeführt wird.

2. Verfahren nach Anspruch 1, dadurch gekennzeichnet, daß als Inhibitor Hydroxylamin, Jodessigsäure oder N-Ethylmaleinimid verwendet werden.

3. Verfahren nach den Ansprüchen 1 oder 2 dadurch gekennzeichnet, daß die Konzentration des Inhibitors zwischen 0,5 und 20 mmol/l liegt.

4. Verfahren zur Bestimmung von Gerinnungsparametern in mindestens zwei verschiedenen Proben
a. wobei eine von Thrombin katalysierte Bildung eines Fibrinclots erfolgt, und die Bildung des Fibrinclots gemessen wird,
b. die Bestimmung in Gegenwart eines Inhibitors für die Aktivierung von Faktor XIII, Faktor XIIIₐ selbst oder die fibrinvernetzende Wirkung von Faktor XIIIₐ durchgeführt wird.
c. das Meßgefäß nach der ersten Bestimmung
- mit einer Lösung versetzt wird, die die gegenseitige Affinität der Fibrinmoleküle aufhebt
- nach dem Auflösen entleert wird und
- einmal oder mehrmals gewaschen wird, vorzugsweise mit destilliertem Wasser
d. Durchführung der Bestimmung eines Gerinnungsparameters in der zweiten Probe in dem genannten Meßgefäß.

5. Verfahren nach Anspruch 4 dadurch gekennzeichnet, daß als Auflösemittel (s. 4c) entweder Säure, Lauge, NaSalicylat + EDTA allein oder in Kombination eingesetzt werden.

6. Verfahren nach einem der Ansprüche 4 und 5 dadurch gekennzeichnet, daß der pH unter 2 oder über 13 liegt falls nur Säure oder Lauge eingesetzt wird.

## Claims

1. Method for the determination of coagulation parameters in sample material during which a thrombin-catalyzed formation of a fibrin clot occurs and the formation of the fibrin clot is measured, wherein the determination is carried out in the presence of an inhibitor of the activation of factor XIII, factor XIIIa itself or of the fibrin cross-linking activity of factor XIIIa.

2. Method as claimed in claim 1, wherein hydroxylamine, iodoacetic acid or N-ethylmaleinimide are used as the inhibitor.

3. Method as claimed in claims 1 or 2, wherein the concentration of the inhibitor is between 0.5 and 20 mmol/l.

4. Method for the determination of coagulation parameters in at least two different samples
a. wherein a thrombin-catalyzed formation of a fibrin clot occurs and the formation of the fibrin clot is measured,
b. the determination is carried out in the presence of an inhibitor of the activation of factor XIII, factor XIIIa itself or of the fibrin cross-linking activity of factor XIIIa
c. after the first determination,
- a solution is added to the measuring vessel which abolishes the mutual affinity of the fibrin molecules
- the measuring vessel is emptied after the dissolution and
- it is washed once or several times, preferably with distilled water.
d. Carrying out the determination of a coagulation parameter in the second sample in the said measuring vessel.

5. Method as claimed in claim 4, wherein either an acid, a base, Na salicylate + EDTA are used alone or in combination as the dissolution agent (see 4c).

6. Method as claimed in one of claims 4 and 5, wherein the pH is below 2 or above 13 if only an acid or a base is used.

## Revendications

1. Procédé de détermination de paramètres de coagulation dans des échantillons, dans lequel se produit une formation d'un caillot de fibrine, catalysée par la thrombine, et dans lequel on mesure la formation du caillot de fibrine, caractérisé en ce que la détermination est effectuée en présence d'un inhibiteur de l'activation du facteur XIII, du facteur XIIIa elle-même, ou de l'action de réticulation de la fibrine du facteur XIIIa.

2. Procédé selon la revendication 1, caractérisé en ce qu'on utilise, à titre d'inhibiteur, de l'hydroxylamine, de l'acide iodoacétique ou du N-éthylmaléinimide.

3. Procédé selon les revendications 1 ou 2, caractérisé en ce que la concentration de l'inhibiteur se situe entre 0,5 et 20 mmoles/l.

4. Procédé de détermination de paramètres de coagulation dans au moins deux échantillons différents, dans lequel
a. se produit une formation d'un caillot de fibrine, catalysée par la thrombine et dans lequel on mesure la formation du caillot de fibrine,
b. la détermination est réalisée en présence d'un inhibiteur de l'activation du facteur XIII, du facteur XIIIa elle-même, ou de l'action de réticulation de la fibrine du facteur XIIIa,
c. après la première détermination, le récipient de mesure
- est mélangé à une solution qui neutralise l'affinité réciproque des molécules de fibrine
- est vidé après la dissolution et
- est lavé une ou plusieurs fois, de préférence avec de l'eau distillée
d. la détermination d'un paramètre de coagulation dans le deuxième échantillon est réalisée dans ledit récipient de mesure.

5. Procédé selon la revendication 4, caractérisé en ce qu'on utilise à titre d'agent de dissolution (voir 4c) un acide, une base, du salicylate de Na + EDTA, soit seuls, soit en combinaison.

6. Procédé selon l'une des revendications 4 et 5, caractérisé en ce que le pH est inférieur à 2 ou supérieur à 13, si on n'utilise que de l'acide ou de la base.
